(19) Europäisches Patentamt
European Patent Office
Office européen des brevets

(11) **EP 1 175 489 B1**

(12) **FASCICULE DE BREVET EUROPEEN**

(45) Date de publication et mention
de la délivrance du brevet:
**31.05.2006 Bulletin 2006/22**

(51) Int Cl.:
*C12N 15/11* (2006.01)     *A61K 31/713* (2006.01)
*A61P 31/14* (2006.01)     *A61P 35/00* (2006.01)

(21) Numéro de dépôt: **00910916.6**

(86) Numéro de dépôt international:
**PCT/FR2000/000586**

(22) Date de dépôt: **09.03.2000**

(87) Numéro de publication internationale:
**WO 2000/053745 (14.09.2000 Gazette 2000/37)**

(54) **OLIGONUCLEOTIDES CONTENANT UNE SEQUENCE ANTISENS STABILISES PAR UNE STRUCTURE SECONDAIRE ET COMPOSITIONS PHARMACEUTIQUES LES CONTENANT**

OLIGONUKLEOTIDE DIE EINE ANTISENSE-SEQUENZ ENTHALTEN DIE DURCH EINE SEKONDÄRSTRUKTUR STABILISIERT SIND UND PHARMAZEUTISCHE ZUSAMMENSETZUNGEN DIE SIE ENTHALTEN

OLIGONUCLEOTIDES CONTAINING AN ANTISENSE SEQUENCE STABILISED BY A SECONDARY STRUCTURE AND PHARMACEUTICAL COMPOSITIONS CONTAINING SAME

(84) Etats contractants désignés:
**AT BE CH CY DE DK ES FI FR GB GR IE IT LI LU MC NL PT SE**

(30) Priorité: **09.03.1999 FR 9902921**

(43) Date de publication de la demande:
**30.01.2002 Bulletin 2002/05**

(73) Titulaires:
• **Bioalliance Pharma (S.A.)**
**75013 Paris (FR)**
• **CENTRE NATIONAL DE LA RECHERCHE SCIENTIFIQUE**
**75016 Paris Cédex (FR)**
• **INSTITUT GUSTAVE ROUSSY -IGR-**
**94805 Villejuif Cedex (FR)**

(72) Inventeurs:
• **MALVY, Claude**
**F-91800 Boussy Saint Antoine (FR)**
• **MAKSIMENKO, Andrei**
**F-75014 Paris (FR)**
• **HELIN, Valérie**
**F-75013 Paris (FR)**
• **GOTTIKH, Marina**
**Moscou, 117593 (RU)**

(74) Mandataire: **Breese, Pierre**
**BREESE DERAMBURE MAJEROWICZ**
**38, avenue de l'Opéra**
**75002 Paris (FR)**

(56) Documents cités:
WO-A-94/01550     WO-A-94/12633
WO-A-94/23026     WO-A-95/29241

• **TANAKA KAZUHIRO ET AL: "EWS-Fli1 antisense oligodeoxynucleotide inhibits proliferation of human Ewing's sarcoma and primitive neuroectodermal tumor cells." JOURNAL OF CLINICAL INVESTIGATION, vol. 99, no. 2, 1997, pages 239-247, XP002140063 ISSN: 0021-9738 cité dans la demande**
• **BARKER RH JR. ET AL.: "Plasmodium falciparum: effect of chemical structure on efficacy and specificity of antisense oligonucleotides against malaria in vitro." EXP PARASITOL 1998 JAN;88(1):51-9, XP000856056 cité dans la demande**
• **TANG, J. ET AL.: "Self-stabilized antisense oligodeoxynucleotide phosphorothioates: properties and anti-HIV activity" NUCLEIC ACIDS RESEARCH., vol. 21, 1993, pages 2729-2735, XP002123468 ISSN: 0305-1048 cité dans la demande**
• **ABE T ET AL.: "Specific inhibition of influenza virus RNA polymerase and nucleoprotein gene expression by circular dumbbell RNA/DNA chimeric oligonucleotides containing antisense phosphodiester oligonucleotides." FEBS LETT 1998 MAR 20;425(1):91-6, XP002123469**

- JOLLÈS B ET AL: "Opening of the extraordinarily stable mini- hairpin d(GCGAAGC)." NUCLEIC ACIDS RESEARCH, (1997 NOV 15) 25 (22) 4608-13., XP002123470
- SOKOL D L ET AL: "Real time detection of DNA.RNA hybridization in living cells." PROCEEDINGS OF THE NATIONAL ACADEMY OF SCIENCES OF THE UNITED STATES OF AMERICA, (1998 SEP 29) 95 (20) 11538-43., XP002123471
- GOTTIKH, M. B. ET AL: ".alpha.-.beta. Chimeric Oligonucleotides Form a New Stable "Snail-like" Structure" J. AM. CHEM. SOC. (1996), 118 (9), 2126-30, XP000644536

LISTE DE SEQUENCES

<110> Bioalliance Pharma S.A.

<120> Oligonucléotides contenant une séquence antisens stabilisés par une structure secondaire et compositions pharmaceutiques les contenant

<130> 2890PCT1

<140> FR99/02921
<141> 1999-03-09

<160> 2

<170> PatentIn Ver. 2.1

<210> 1
<211> 29
<212> ADN
<213> Séquence artificielle

<220>
<223> Description de la séquence
artificielle:oligonucléotides antisens

<220>
<221> misc_feature
<222> (1)..(29)
<223> Chaque nucléotide de la séquence est un phosphorothioate

<400> 1
tgagtcataa gaagggttct gctgcccgt 29

<210> 2
<211> 30
<212> ADN
<213> Séquence artificielle

<220>
<223> Description de la séquence
artificielle:oligonucléotides antisens

<220>
<221> misc_feature
<222> ()..(19)
<223> Les nucléotides occupant une position de 12 à 19 sont des phosphorothioates

<400> 2
gtagcgaagg gttctgctgc ccgtagctgc 30

## Description

La présente invention concerne des compositions pharmaceutiques comprenant des oligonucléotides capables de s'hybrider avec une séquence d'acide nucléique cible, et qui sont stabilisés par une structure secondaire de manière à être résistant à la dégradation dans des milieux biologiques, principalement à la dégradation par des nucléases.

Les acides nucléiques anti-sens sont des séquences nucléiques capables de s'hybrider sélectivement avec des ARNs messager cellulaires-cible pour inhiber leur traduction en protéine. Ces oligonucléotides forment avec l'ARNm-cible, de manière locale, des régions double brin, par interaction de type Watson-Crick classique.

Beaucoup d'états pathologiques sont la conséquence de l'expression d'un gène anormal au sein d'une cellule. De tels gènes étrangers peuvent s'intégrer dans l'ADN cellulaire, après une infection virale par exemple, et peuvent donc être exprimés par la cellule. Il en est de même pour de nombreux gènes oncogènes, qui sont capables de conférer le phénotype cancéreux à une cellule eucaryote, et une tumeur dans un organisme entier.

Une des approches proposées dans l'art antérieur pour inhiber l'action de tels gènes réside dans l'utilisation d'oligonucléotides antisens (1-3). La régulation de l'expression de gène-cibles au moyen d'oligonucléotides antisens constitue en effet une approche thérapeutique en développement croissant. Cette approche repose sur la capacité des oligonucléotides à s'hybrider spécifiquement à des régions complémentaires d'un acide nucléique, et à inhiber ainsi spécifiquement l'expression de gènes déterminés. Cette inhibition peut intervenir soit au niveau traductionnel (oligonucléotide anti-sens) soit au niveau transcriptionnel (oligonucléotide anti-gène).

L'application thérapeutique d'une technologie antisens est largement étudiée dans de nombreuses infections virales, incluant le virus d'immunodéficience acquise (4), le virus de l'influenza (5), le virus d'Epstein-Barr (6), les papillomavirus humains (7,8) et le virus simplex de l'herpes (9,10).

Il peut s'agir par exemple d'oligonucléotides synthétiques, de petite taille, complémentaires d'ARNm cellulaires, et qui sont introduits dans les cellules-cible. De tels oligonucléotides ont par exemple été décrit dans la demande de brevet européen No. 92 574. Il peut également s'agir de gènes anti-sens dont l'expression dans la cellule cible génère des ARN complémentaires d'ARNm cellulaires. De tels gènes ont par exemple été décrits dans la demande de brevet européen No. 140 308.

Cependant l'utilisation *in vivo* d'acides nucléiques antisens se heurte à un certain nombre de difficultés qui limitent jusqu'à aujourd'hui leur exploitation thérapeutique.

En effet, les acides nucléiques présentent une grande sensibilité à la dégradation par des enzymes de l'organisme, telles que les nucléases (11,12), ce qui implique l'emploi de doses importantes. De plus, ils ont une faible pénétration dans certains types cellulaires et une distribution intracellulaire souvent inadéquate, ce qui les rend sans effet thérapeutique. Enfin, il est important de pouvoir disposer de séquences suffisamment sélectives et stables pour obtenir un effet spécifique sans altérer d'autres fonctions cellulaires.

Depuis les premières tentatives par Stephenson et Zamecnik (13) pour inhiber le virus du sarcome de Rous en utilisant des oligonucléotides phosphodiesters, de nombreux efforts ont été fait pour optimiser l'efficacité de ces oligonucléotides, notamment sur la pénétration cellulaire (14-16), la fixation sur leur cible (17), la résistance aux nucléases (18-20).

Le problème de la résistance des oligonucléotides aux nucléases demeure un problème limitant les possibilités de développement de cette stratégie thérapeutique. Pour tenter de résoudre ce problème, il a été proposé dans l'art antérieur de modifier chimiquement le squelette phosphodiester des acides nucléiques pour donner lieu à de nouvelles classes d'oligonucléotides artificiels (21-23). Parmi ceux-ci, on peut citer les oligonucléotides phosphonates, phophoramidates et phosphorothioates qui sont décrit par exemple dans la demande de brevet internationale PCT No. WO94/08003, ou les oligonucléotides couplés à différents agents tels que du cholestérol, un peptide, un polymère cationique, etc....

Toutefois, si certains de ces oligonucléotides modifiés présentent une bonne résistance aux nucléases, ces modifications peuvent présenter l'inconvénient d'être accompagnées par la perte d'autres propriétés importantes pour l'activité antisens, tel que leur affinité pour les cibles d'ARN, leur capacité à moduler la dégradation des ARNs par les Rnases, et leur pouvoir de pénétration et de distribution dans les différents compartiments cellulaires reste très faible (1,22,24). De plus, leur activité biologique n'est pas toujours augmentée et ils peuvent présenter certains effets secondaires liés à la présence de motifs non-naturels dans leur structure. En effet, les oligonucléotides ainsi modifiés présentent des caractéristiques indésirables comme des interactions non-spécifiques avec des protéines cellulaires, et une grande cytotoxicité (25-29).

Une autre méthode permettant d'accroître la résistance des oligonucléotides aux nucléases, mais utilisant des phosphodiesters naturels, consiste à greffer sur l'extrémité 3' de la séquence à protéger un conjugué de dodecanol (demandes de brevet européen No. EP 117 777 et No. EP 169 787).

Pour palier les inconvénient rapportés précédemment, il a aussi été proposé dans l'art antérieur, par exemple dans la demande de brevet internationale PCT No. WO 94/12633, d'adjoindre à l'une et/ou l'autre des extrémités de la séquence antisens à protéger des séquences nucléotidiques dont la structure secondaire se présente sous la forme de boucles ou d'épingles à cheveux, capable d'empêcher les nucléases de venir dégrader la séquence antisens (30-35). Or, cette technique n'est pas satisfaisante car la présence de nucléotides supplémentaires des séquences en épingles à cheveux gênent l'hybridation de la séquence antisens avec les acides nucléiques cibles.

La présente invention vise précisément à offrir de nouveaux oligonucléotides capables de modifier ou d'inhiber l'expression de gènes *in vivo* ou *in vitro* et résistant aux digestions nucléasiques sans présenter les inconvénients décrits ci-dessus.

L'invention concerne une composition pharmaceutique pour le traitement du sarcome d'EWING, caractérisée en ce qu'elle comprend à titre de principe actif l'un au moins des oligonucléotides représentés dans la liste de séquence sous les numéros SEQ ID NO.1 ou SEQ ID NO.2.

L'invention concerne également les compositions pharmaceutiques contenant un ou plusieurs oligonucléotides décrits précédemment identiques ou différents associés dans la dite composition à un véhicule pharmaceutiquement acceptable. Les oligonucléotides de l'invention peuvent être libres, encapsulés, couplés, conjugués à des substances diverses comme des anticorps, des protéines, des liposomes, des microsphères, des microorganismes ou des cellules adaptés aux modes d'administration utilisés, ou à tout autre type de vecteur comme des nanoparticules, des dendrimères, des lipides cationiques ou des peptides.

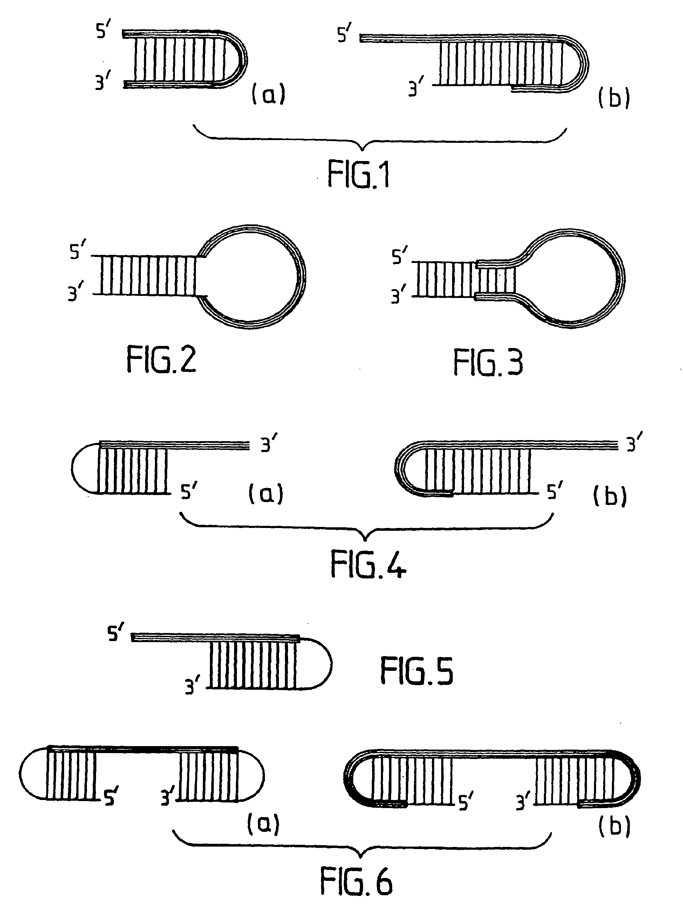

D'autres avantages et caractéristiques de l'invention apparaîtront des exemples qui suivent et de des figures 1 à 6 représentant schématiquement des exemples de réalisation des oligonucléotides de l'invention. Dans ces figures, les traits épais symbolisent la séquence antisens, les traits fins symbolisent la ou les séquences supplémentaires si elle (s) est(sont) présente(s), et les barres horizontales symbolisent les liaisons de la paire de bases.

Exemple 1 : Étude d'oligonucléotdes antisens de l'invention comprenant une région complémentaire de la région d'initiation de la traduction de l'ARN *env* du rétrovirus de la leucémie murine de Friend.

Il sera fait référence dans cet exemple aux dessins en annexes dans les lesquels :

- La figure 7 représente les oligonucléotides utilisés dans la partie expérimentale ci-après. Les séquences cibles d'ADN et d'ARN de 21 mer sont soulignées. Les séquences des oligodéoxyribonucléotides antisens complémentaires des cibles D et R sont en caractères gras.
- La figure 8 représente l'analyse en électrophorèse de la formation de duplexes en PAGE natif à 15% à 37°C. En A : fixation de la cible d'ARN marqué au $^{32}$P (ligne 1) aux oligodéoxynucléotides 21L (ligne 2), 21PS (ligne 3), H6 (ligne 4), H8 (ligne 5), H0 (ligne 6), Dh6 (ligne 7), L8 (ligne 8), L10 (ligne 9), SL (ligne 10), 55L (ligne 11). En B : : fixation de la cible d'ADN marqué au $^{32}$P (ligne 1) aux oligodéoxynucléotides 21L (ligne 2), 21PS (ligne 3), H6 (ligne 4), H8 (ligne 5), H0 (ligne 6), Dh6 (ligne 7), L8 (ligne 8), L10 (ligne 9), SL (ligne 10), 55L (ligne 11).
- La figure 9 représente le clivage de la cible d'ARN par la Rnase H en présence des oligonucléotides antisens 21L (ligne 2), 21PS (ligne 3), H6 (ligne 4), H8 (ligne 5), H0 (ligne 6), Dh6 (ligne 7), L8 (ligne 8), L10 (ligne 9), SL (ligne 10), 55L (ligne 11) . ARN R marqué au $^{32}$P, seul (ligne 1) et en présence de Rnase H sans ODNS (ligne 12). Les flèches indiquent les sites majeurs de clivages.
- La figure 10 représente la dégradation des oligonucléotides antisens dans le DMEM supplémenté avec 10% de FBS inactivé par la chaleur, en l'absence (a, b) et en présence (c, d) de SuperFect™.
- La figure 11 représente la dégradation des oligonucléotides antisens dans les lysats cellulaires : (a, b) lysat HeLa, (c, d) lysat NIH 3T3.
- La figure 12 représente la dégradation des oligonucléotides antisens complexés avec SuperFect™ à l'intérieur des cellules : (a, b) HeLa, (c, d) NIH 3T3.

I - Matériels et méthodes.

1) Oligonucléotides.

Les oligonucléotides ont été acquis auprès de la Société Eurogentec (Seraing, Belgique), puis désalés sur des colonnes Sephadex G25 et quantifiés par absorbance à 260 nm.

2) Protection de l'extrémité 5' phosphate des oligonucléotides antisens.

Le marquage à l'extrémité 5' des oligonucléotides a été réalisé en utilisant de l'ATP($^{32}$P-γ) (Amersham) et la T4 polynucléotide kinase (Promega). Les oligonucléotides marqués ont été purifiés par électrophorèse sur un gel de polyacrylamide dénaturant à 20%, puis dissous dans 100 μl de tampon de N-méthylmorphine 1 M (pH 7,5), MgCl$_2$ 20 mM, contenant 50 % d'éthanol et la solution a été supplémentée par 10 mg d 1-éthyl-3(3'-diméthylaminopropyl)carbodiimide. La réaction a été réalisée pendant 16 h à 4°C et les oligonucléotides ont été précipités deux fois avec 1 ml d'une solution de LiClO$_4$ à 2% dans de l'acétone et lavés avec de l'acétone. Ces oligonucléotides ayant le phosphate terminal protégé par un résidu éthyle contre l'hydrolyse par une phosphatase ont été ensuite mélangés avec des oligodéoxynucléotides (ODNs) non marqués et utilisés pour étudier leur résistance à une digestion enzymatique.

### 3) Expériences de dénaturation thermique.

Les courbes d'aborbance/température ont été enregistrées à 260 nm en utilisant un spectrophotomètre Uvikon 933 équipé d'un thermoprogrammateur. Les solutions d'ODNs ont été préparées dans 600 $\mu$l d'un tampon phosphate de sodium 10 mM (pH 7,5) avec NaCl 50 mM. La concentration de chaque brin d'oligonucléotide est de 10$^{-6}$ M. L'absorbance a été mesurée pendant que la température était augmentée de 20°C à 80°C à raison de 0,5°C par minute. Les températures de fusion (T$_m$) ont été déterminées par ajustement informatique à partir de la dérivée première de l'absorbance suivant 1/T. La précision des T$_m$ a été estimée à $\pm$ 0,5°C à partir des répétitions des expériences. Les valeurs d'énergie libre pour la dissociation du duplexe ont été dérivées par ajustement informatique des courbes de fusion en utilisant le model à deux-états (36).

### 4) Gel d'électrophorèse natif.

Les matrice ARN et ADN (R et D) ont été marquées en utilisant l'ATP($^{32}$P-$\gamma$) et la polynucléotide kinase de T4. Les oligonucléotides (10 pmoles pour chaque brin) ont été dissous dans 10 $\mu$l de tampon Tris-acétate (pH 7,5), CH$_3$COONa 150 mM, MgCL$_2$ 2 mM, et incubés à 37°C pendant 1 heure puis supplémentés avec 1 $\mu$l d'une solution à 70% de glycérol contenant du cyanol xylène et du bleu de bromophénol. Les éléctrophorèses ont été réalisés dans un gel d'acrylamide à 15% non dénaturant (19:1 acrylamide/bis-acrylamide) dans le même tampon Tris-acétate à 37°C pendant 24 heures (9 V/cm).

### 5) Clivage par la Rnase-H.

Afin d'étudier le déclenchement de l'activité RNase H par les ODNs, 10 pmoles de ceux-ci ont été mélangés avec 1 pmole de la matrice d'ARN (R) marqué en 5'au ($^{32}$P) dans 10 $\mu$l de tampon Tris-HCl 20 mM (pH 7,5), MgCl$_2$ 10 mM; KCl 100 mM, DTT 0,1 mM en présence de 0,5 $\mu$l de RNasin (Gibco BRL) et incubés pendant 30 minutes à 37°C. Puis 0,5 U de RnaseH d'E. coli (Promega, Madison, WI) a été ajouté et les mélanges ont été incubés à 37°C pendant 15 minutes. Les échantillons ont été précipités avec de l'acétone contenant 2 % de LiClO$_4$, séchés, dissous dans 4 $\mu$l de formamide:eau (4:1), 0,01 % de bleu de bromophénol, et 0,01 % de cyanol de xylène et analysés par éléctrophorèse dans un gel de polyacrylamide à 20% dénaturant suivie d'une autoradiography.

### 6) Cellules et milieux.

Des lignées cellulaires NIH 3T3 et HeLa ont été cultivées dans un milieu DMEM supplémenté avec respectivement 5% et 10% de sérum bovin foetal inactivé par la chaleur (FBS) (Gibco, BRL), de la streptomycine (100 mg/$\mu$l) et de lapenicilline (100 U/ml). Toutes les cellules ont été incubées à 37°C dans 5% CO$_2$.

### 7) Préparation des lysats de cellules.

Les cellules NIH 3T3 et HeLa ont été lavées trois fois avec du PBS puis déposées dans 1 ml de tampon Sodium-Phosphate 10 mM (pH 7,5), MgCl$_2$ 10 mM, NaCl 150 mM, DTT 1 mM, 1% NP-40, 02 mg/ml phénylméthylsulphonylfluoride (PMSF) et conservées à -20°C pendant 30°C. Après décongélation, les cellules ont été centrifugées à 14000 g pendant 15 minutes à 4°C. Le surnageant a été utilisé pour étudier la dégradation enzymatique des oligonucléotides. La concentration en protéine dans chaque lysat a été quantifiée en utilisant la BSA comme standard (37).

### 8) Étude de la dégradation de l'oligonucléotide dans un milieu biologique.

La détermination du taux de dégradation de l'oligonucléotide a été effectuée dans du DMEM contenant 10% de FBS inactivé par la chaleur (56°C pendant 30 minutes) et dans les lysats de cellules. Les lysats ont été dilués dans un tampon sodium-phosphate 10 mM (pH 7,5), MgCl$_2$ 10 mM, NaCl 150 mM, DTT 1 mM afin de disposer de la même concentration totale de protéine de 1,22 mg/ml. Pour éviter le clivage enzymatique du phosphate marqué au $^{32}$P, les oligonucléotides avec le phosphate terminal protégé préparés comme décrit précédemment ont été utilisés. Les ODNs marqués au $^{32}$P à une concentration de 10 $\mu$M ont été incubés dans 120 $\mu$l du milieu correspondant à 37°C. A différents temps, des aliquots de 15 $\mu$l ont été prélevés, supplémentés par 15 $\mu$l d'EDTA 50 mM et congelés à -20°C. Les échantillons ont été extraits deux fois avec un mélange phénol/chloroforme/iso-amyl alccol (25/24/1). Les oligonucléotides ont été précipités à partir des fractions aqueuses par 10 volumes d'acétone contenant 2% de $LiClO_4$, séchés, et dissous dans 5 $\mu$l d'un mélange formamide/eau (4/1), 0,01% de bleue de bromophénol, et 0,01% de cyanol xylène. Les échantillons ont été analysés par électrophorèse sur un gel de polyacrylamide 20% dénaturant. Les gels obtenus ont été scannés en utilisant un phosphoimager (Storm 840, Molecular Dynamics). La dégradation des oligonucléotides a été quantifiée comme le rapport du signal efficace des bandes correspondant aux oligonucléotides intacts et dégradés. La précision des pourcentage de dégradation a été estimée à $\pm$ 0,5% sur la base des répétitions des expériences.

9) Étude de la dégradation de l'oligonucléotide dans la cellule.

Un jour avant, les cellules ont été ensemencées sur une plaque 6 puits pour obtenir 60-80% de confluence ($4x10^5$ cellules). 5 $\mu$g de chaque ODN ont été mélangés avec 6 $\mu$l de SuperFect™ (Quiagen, Canada) dans un volume final de 150 $\mu$l de DMEM (sans FBS et antibiotique) pendant 10 minutes à la température de la pièce. Les cellules ont été lavées avec du PBS, les mélanges de SuperFect™-ODN ont été dilués avec 850 $\mu$l de 10 % (pour les cellules HeLa) ou 5% (pour les cellules NIH 3T3) FBS DMEM (avec des antibiotiques) et ajoutés aux cellules.

Le surnageant a été extrait après 16 ou 48 heures, et les cellules ont été récoltées par un traitement à la trypsine. Ensuite, les cellules ont été lavées trois fois avec du PBS, suspendues dans 500 $\mu$l de tampon sodium-phosphate 10 mM (pH 7,5), NaCl 150 mM, EDTA 20 mM, 1% NP-40 et laissées 30 minutes à -20°C. Après décongélation, les cellules ont été chauffées 30 minutes à 90°C afin de détruire complètement tous les compartiments cellulaires et extraites deux fois avec un mélange phénol/ chloroforme/ alcool iso-amyl (25/24/1). Les ODNs ont été précipités à partir des fractions aqueuses contenant 0,5 M d'acétate de sodium en ajoutant de l'éthanol 5 fois en excès, dissous dans un mélange formamide/eau (4/1), 0,01% de bleu de bromophénol et 0,01% de cyanol de xylène et analysés par électrophorèse sur gel dénaturant 20%. Les gels obtenus ont été scannés en utilisant un phosphorimager (Strom 840, Molecular Dynamics) La dégradation des oligonucléotides a été quantifiée comme un rapport du signal efficace des bandes correspondant aux oligonucléotides intacts et dégradés.

II - Résultats.

1) Structures des oligonucléotides structurés.

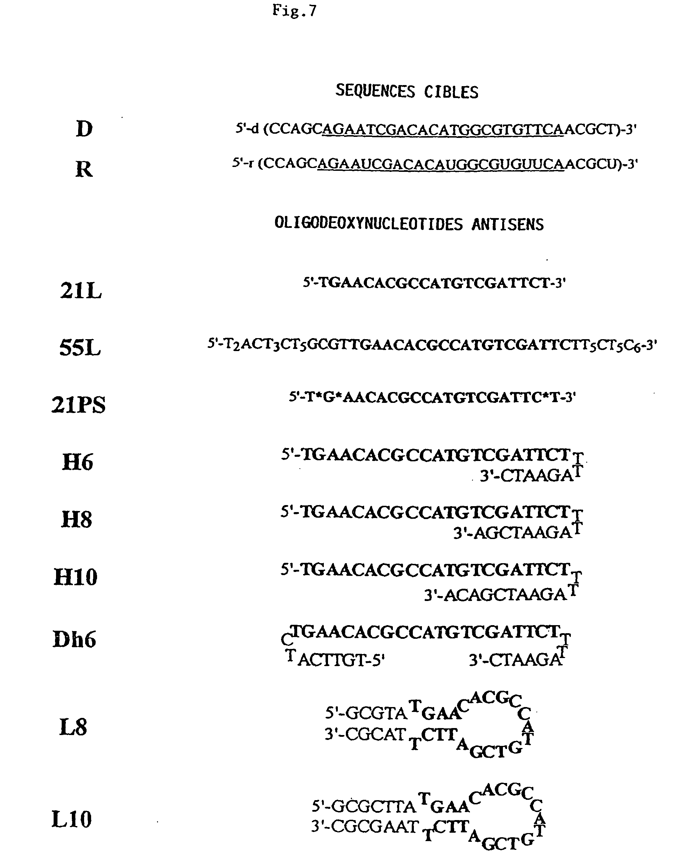

Comme montré à la figure 7, tous les oligodéoribonucléotides étudiés comprennent la séquence de 21 bases 5'-TGAACACGCCATGTCGATTCT-3' soulignée ou indiquée en gras dans la figure 7, complémentaire de la région d'initiation de la traduction de l'ARN env du rétrovirus de la leucémie murine de Friend. Les oligonucléotides 21L et 55L ont une structure linéaire comme l'oligonucléotide 21PS. Toutefois, ce dernier contient deux groupes phosphothioates à chaque extrémité pour le protéger de la dégradation enzymatique. Les oligonucléotides H6, H8 et H10 présentent un nombre différent de paires de bases (pb) dans la séquence supplémentaire de la structure secondaire en épingle à cheveux localisée à l'extrémité 3'. DH6 peut former une structure secondaire en épingle à cheveux avec des queues de 6pb aux extrémités 5' et 3'. L8 et L10 peuvent former des structures secondaires en boucle avec 13 nucléotides situés dans la boucle et dans les queues avec respectivement 8 et 10 pb.

2) Étude des propriétés de liaison des oligonucléotides.

Le tableau 1 ci-dessous rapporte la température de fusion ($T_m$) et le $\Delta G^0_{37}$ déterminés à la fois pour les oligonucléotides (ONs) seuls et pour les complexes avec les cible ADN (D) ou ARN (R), dans 10 mM phosphate, 50 mM NaCl, pH = 7,5.

<u>Tableau 1</u>

| ONs + cible | $T_m$ (°C) [a] | $-\Delta H$ [b] (kcal mol$^{-1}$) | $-\Delta S$ (e.u) [c] | $-\Delta G^u_{37}$ (kcal mol$^{-1}$) |
|---|---|---|---|---|
| 21L + D | 60 | 101 | 267 | 18,2 |
| 21L + R | 57 | 105 | 286 | 16,3 |
| 21PS + D | 60 | 82 | 215 | 15,4 |
| 21PS + R | 57 | 86 | 232 | 14,1 |
| H6 | 52 | 45 | 139 | 1,9 |
| H6 + D | 58 | 92 | 249 | 14,8 |
| H6 + R | 56 | 82 | 222 | 13,2 |
| H8 | 66 | 62 | 190 | 3,1 |

Suite de tableau

| ONs + cible | $T_m$ (°C) [(a)] | $-\Delta H$ [(b)] (kcalmol$^{-1}$) | $-\Delta S$(e.u)[(c)] | $-\Delta G^u_{37}$ (kcal mol$^{-1}$) |
|---|---|---|---|---|
| H8 + D | 60 | 60 | 151 | 13,2 |
| H8 + R | 56 | 52 | 130 | 11,7 |
| H10 | 75 | 44 | 128 | 4,3 |
| H10 + D | 43, 60, 73 | 43 | 102 | 11,4 |
| H10 + R | 40, 57, 73 | 35 | 78 | 10,8 |
| Dh6 | 52 | 35 | 104 | 2,8 |
| Dh6 + R | 57 | 90 | 245 | 14,1 |
| Dh6 + R | 55 | 80 | 218 | 12,4 |
| L8 | 47 | 46 | 142 | 2,0 |
| L8 + D | 60 | 96 | 260 | 15,4 |
| L8 + R | 57 | 101 | 280 | 14,2 |
| L10 | 53 | 68 | 208 | 3,5 |
| L10 + D | 57 | 94 | 255 | 15,0 |
| L10 + R | 55 | 96 | 265 | 13,9 |

$\Delta H$, $\Delta S$ et $\Delta G$ sont les moyennes de plusieurs valeurs obtenues de courbes de fusion indépendantes et sont arrondis.

(a) : l'erreur sur les $T_m$ est de $\pm$ 0,5°C.

(b) : l'erreur sur les $\Delta H$ est de $\pm$ 5 kcalmol$^{-1}$.

(c) : l'erreur sur les $\Delta S$ est de $\pm$ 10 e.u.

Les domaines double brin des ODNs possédant une structure secondaire, aussi désignés "SODNs) ont été trouvés stables dans des conditions sensiblement physiologiques. La stabilité thermique des duplexes internes dans les ODNs H6, H8, H10 et L8 et L10 dépend du nombre de paires de base dans leur région de queue. Toutefois, comme montré dans le tableau 1, tous les SODNs sont capables d'interagir avec à la fois les cibles d'ADN et d'ARN pour former des duplexes intermoléculaires ayant des paramètres thermodynamiques différents des duplexes intramoléculaires. Le $T_m$ trouvé pour ces duplexes bimoléculaires diffère significativement du $T_m$ des SODNs et correspond approximativement au $T_m$ détecté pour les duplexes formés par l'ODN linéaire 21 L avec les deux cibles. La seule exception est l'oligonucléotide H10 qui présente une épingle à cheveux très stable ($T_m$ = 75°C). En présence de cibles D ou R, la courbe de fusion présente trois périodes :

- la première correspond à la fusion d'un duplexe intermoléculaire partiel formé entre le fragment simple brin de 11 mers de cet ODN et la cible (43°C avec la cible d'ADN et 40°C avec la cible d'ARN),
- la seconde correspond à la fusion du duplexe intermoléculaire complet (60°C avec la cible d'ADN et 57°C avec la cible d'ARN),
- la troisième correspond à la fusion de son propre domaine en double hélice (73°C).

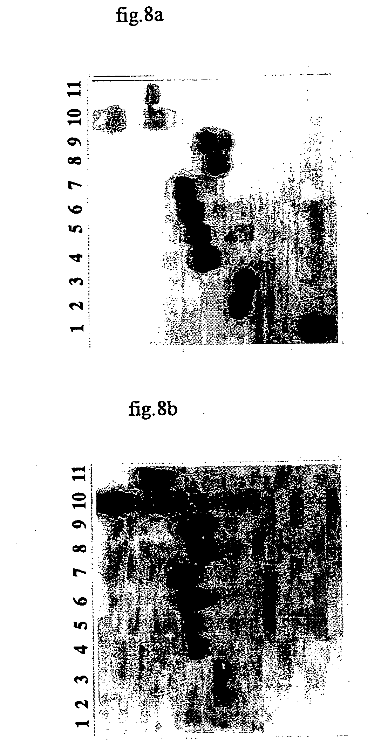

La fixation des SODNs avec les cibles d'ADN et d'ARN a aussi été étudiée par un test de mobilité sur gel. Les ODNs ont été incubés avec des cibles D et R marquées au ($^{32}$P) à 37°C et la mobilité des complexes formés a été déterminée par électrophorèse dans un gel de polyacrylamide non dénaturant à 15%. La figure 8 montre que la mobilité électrophorétique des deux cibles change en présence de tous les SODNs du fait de leur implication dans la formation des complexes correspondants.

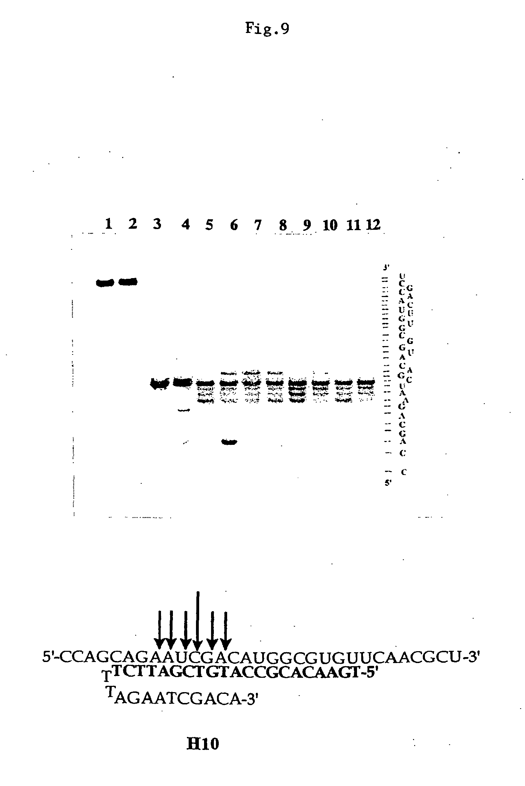

La capacité des SODNs à s'hybrider au brin d'ARN complémentaire et à déclencher la coupure par la Rnase H a aussi été étudiée. La figure 9 montre le résultat de l'incubation de la cible d'ARN (0,1 $\mu$M) et de différents ODNs (1 $\mu$M) avec 0,5 unité de Rnase H pendant 15 minutes à 37°C. La coupure de l'ARN (R) par la RNase H en présence de l'oligonucléotide linéaire 21L et de tous les SODNs intervient avec la même efficacité et aux mêmes sites. Les sites majeurs de coupure sont indiqués par des flèches sur la figure 9. En l'absence d'ODNs complémentaires aucune coupure de l'ARN n'est observée.

Tous ces résultats montrent que la structure double brin interne des ODNs n'empêche pas leur interaction avec leur cibles d'ADN ou d'ARN. Les duplexes internes semblent se dissocier lorsque les complexes bimoléculaires entre les SODNs et leurs cibles sont formés. Évidemment, comme montré dans le tableau 1, les duplexes bimoléculaires sont thermodynamiquement préférés.

3) Stabilité des oligonucléotides antisens vis-à-vis de la dégradation nucléolytique.

La capacité des ODNs avec une structure secondaire à résister à l'hydrolyse enzymatique a été étudié dans du DMEM supplémenté avec 10% de FBS inactivé par la chaleur, généralement utilisé pour la croissance des cellules, ainsi que dans les lysats cellulaires de deux types ce lignées cellulaires (NIH 3T3 et HeLa) Afin d'éviter le clivage du phosphate marqué au ($^{32}$P), des oligonucléotides avec un phosphate protégé en 5'par un résidu éthyl ont été utilisés pour cette étude.

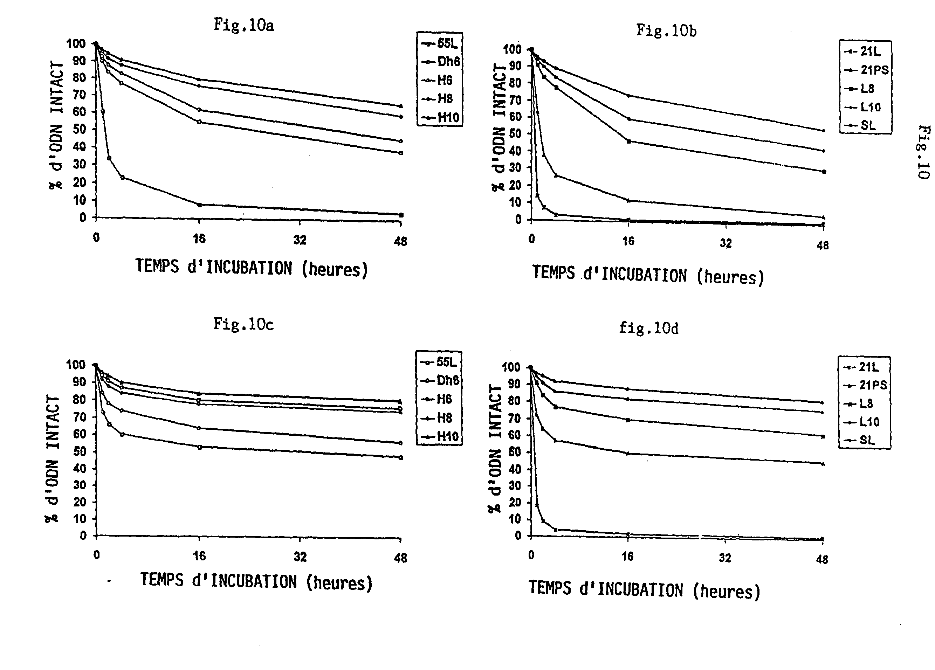

La figure 10 (a) et (b) se rapportant à l'analyse de la dégradation de l'ODN dans le milieu de culture contenant du FBS démontre que la structure secondaire, en épingle à cheveux, en boucle ou en escargot augmente significativement la résistance aux nucléases des SODNs. La résistance des ODNs avec l'épingle à cheveux et la boucle à la dégradation dépend du type de duplexes internes de leur stabilité thermique. Par exemple, la demi-vie de l'épingle à cheveux la plus thermostable H10 est supérieure à celle des épingles à cheveux H6 et H8. En même temps, l'ODN L10 avec une boucle est aussi résistant à la dégradation que l'ODN H8 avec une épingle à cheveux alors que sa stabilité thermique est inférieure (53°C pour L10 et 60°C pour H8). Tous les oligonucléotides linéaires 21L, 55L et 21PS sont rapidement dégradés. Ces résultats confirment les données rapportées précédemment sur la demi-vie très courte des oligonucléotides phophorothoates et phosphodiesters linéaires dans le sérum (10, 11).

Afin d'augmenter la pénétration de l'oligonucléotide dans les cellules plusieurs réactifs de transfection peuvent être utilisés (38-42). L'influence de l'un d'entre-eux, une molécule dendrimérique dénommée SuperFect™, sur la stabilité de l'ODN dans le DMEM supplémenté avec 10% de FBS a été étudiée. Comme montré à la figure 10 (c) et (d), les complexes formés par les ODNs avec SuperFect™ présentent une plus forte résistance à la dégradation nucléolytique que les ODNs seuls. L'augmentation de la résistance est la plus significative avec les ODNs linaires 55L et 21PS. Par exemple, la demi-vie de l'ODN phosphorothioate 21PS avec et sans SuperFect™ est respectivement d'environ 12 heures à 30 minutes. La même augmentation de la stabilité a été observée avec le 55L comme montré à la figure 10 (a) et (c). Ceci est en accord avec les données connues de la littérature montrant que la formation de complexes de composés polyamines avec les ODNs augmente leur stabilité vis-à-vis de nucléases (38). Par ailleurs, la demi-vie de l'ODN non modifié 21L avec et sans SuperFect™ est sensiblement identique. Il est possible que le complexe formé par le SuperFect™ avec cet ODN court ne protège pas suffisament les extrémités de l'oligonucléotide qui subissent les dégradations enzymatiques.

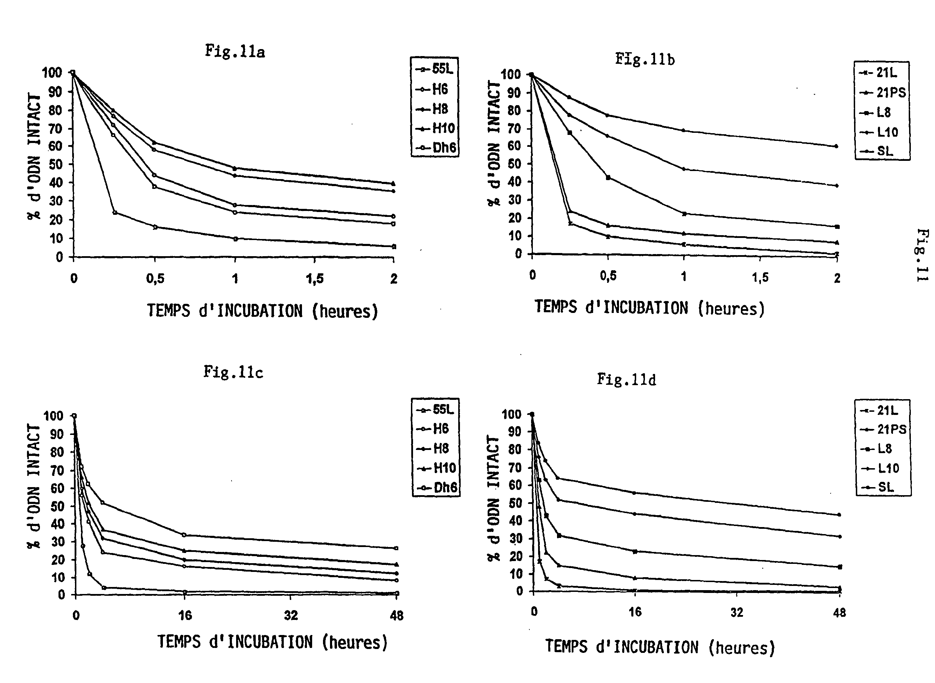

La stabilité des ODNs dans les lysats des cellules HeLa et NIH 3T3 varie avec le type de lysat. Il convient de noter que la concentration en protéine dans ces lysats est identique. En conséquence, les distinctions entre leurs activités nucléolithiques dépend de leur différence quantitative ou qualitative de l'activité des nucléases. La figure 11 (a) et (b) montre que la dégradation est plus rapide dans le lysat HeLA. Tous les ODNs linéaires, 21L, 21PS et 55L, sont rapidement dégradés, leur demi-vie étant d'environ 10 minutes.

En ce qui concerne le lysat NIH 3T3, la figure 11 (c) et (d) montre que le taux de dégradation des SODN est plus faible que dans le lysat HeLa. De façon intéressante, les SODNs Dh6, L8 et L10 avec tous une protection 5' et 3' sont plus stables que les SODNs H6 à H10 qui ont seulement une épingle à cheveux à l'extrémité 3'. Ces résultats suggèrent que la contribution de l'activité exonucléasique 5' sur la dégradation de l'ODN est très importante dans ce lysat. Toutefois, la modification par un phosphorothioate terminal n'a pas d'influence significative sur la résistance de l'ODN, par rapport aux ODNs 21L et 21PS.

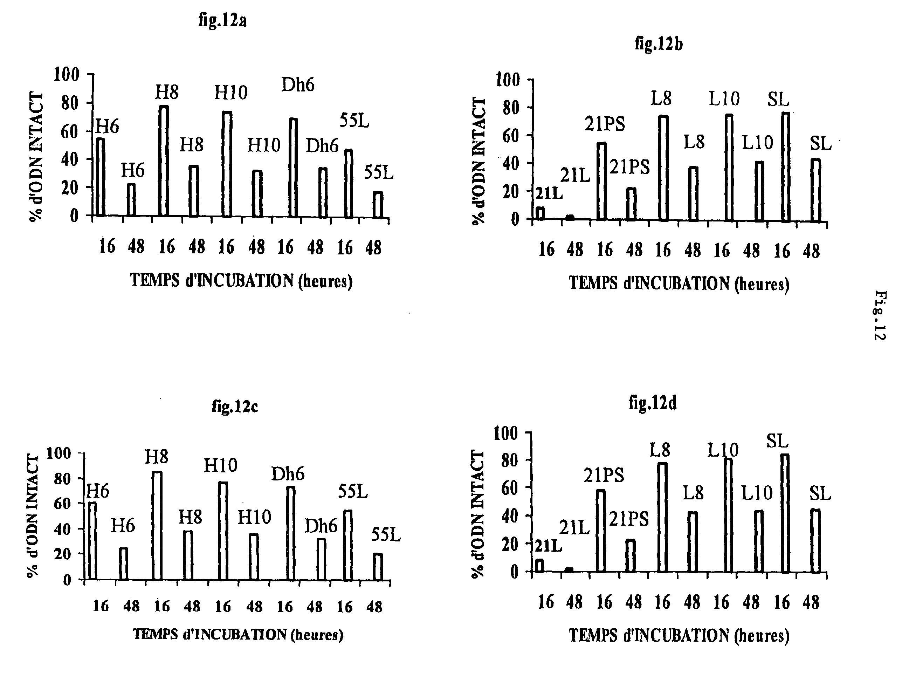

La figure 12 rapporte le evenir des ODNs dans les deux lignées cellulaires HeLA et 3T3. Afin d'améliorer la pénétration des ODN dans les cellules, leurs complexes avec l'agent de transfection TransFect™ ont été formés et incubés avec les cellules. La figure 12 (b) et (d) montre que seulement des traces du 21L ont été trouvées dans les deux types cellulaires après 16 heures d'incubation. Au contraire, la figure 12 (a) à (d) montre que des quantités significatives (50 à 80%) des autres ODNs incluant les linéaires (55L et 21PS) sont détectées dans les cellules. Après 48 heures d'incubation, la quantité d'ODNs intact à l'intérieur des cellules diminue mais demeure à un niveau de 20 à 30%. La figure 12 (a), (b) et (c), (d) montre qu'il n'y a pas de différence significative entre les deux lignées cellulaires. Tous les ODNs avec une structure secondaire ont la même stabilité qui est supérieure à la stabilité des ODNs linéaires 55L et 21PS.

Exemple 2 : Inhibition de l'expression protéique du gène rapporteur pEGFP-N1.

Cet exemple rapporte les résultats du test β-gal/pEGFP-N1 sur cellules HeLa.

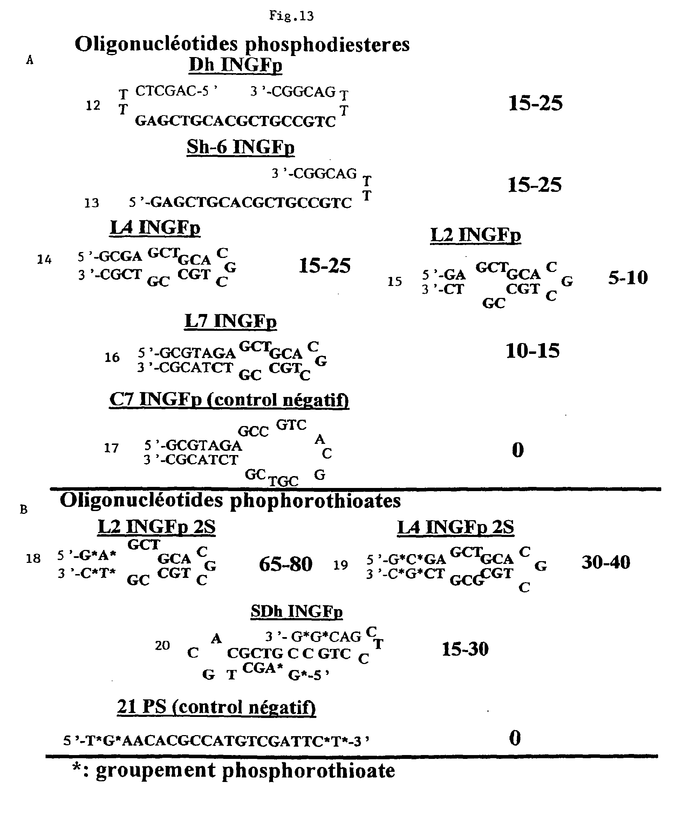

Le tableau 2 ci-dessous indique les caractéristiques des oligonucléotides utilisés dans cett exemple et dont les séquences sont rapportés dans la figure 13 en annexe.

Tableau 2

| Oligonucléo tide | Tm, °C, 50mM NaCl,10mM phosphate (pH 7,0) | Tm, °C, 150mM NaCl, 5mM MgC12, 10mM Tris-HCl (pH 7,0) | Structure possible à 37°C |
|---|---|---|---|
| Dh INGFp | 60 | 71 | Epingle à cheveux |
| Sh-6 INGFp | 56 | 68 | Epingle à cheveux |
| L2 INGFp | 50 | 56 | Tige-boucle |
| L2 INGFp 2S | 48 | 56 | Tige-boucle |
| L4 INGFp 2S | 55 | 63 | Tige-boucle |
| SDh INGFp | 32,45 | 58 | Epingle à cheveux |
| L4 INGFp | 56 | 64 | Tige-boucle |
| L7 INGFp | 52 | 60 | Tige-boucle |
| C7 INGFp | 50 | 57 | Tige-boucle |
| 21 PS | - | 30 | Linéaire |

Le présent exemple rapporte également les résultats obtenus en introduisant dans trois oligonucléotides antisens de l'invention des nucléotides phosphorothiates marqués par des * dans la figure 13.

Le tableau 3 ci-dessous rapporte l'inhibition de l'expression protéique du gène rapporteur pEGFP-N1 (en %) par les oligonucléotides phosphodiesters et phosphorothioates.

Tableau 3

| Oligonucléotides phosphodiesters | (%) | Oligonucléotides Phosphorothioates | (%) |
|---|---|---|---|
| Dh INGFp | 15-25 | | |
| Sh-6 INGFp | 15-25 | | |
| L2 INGFp | 5-10 | L2 INGFp 2S | 65-80 |
| L4 INGFp | 15-25 | L4 INGFp 2S | 30-40 |
| L7 INGFp | 10-15 | | |
| C7 INGFp (contôle négatif) | 0 | 21 PS (contôle négatif) | 0 |
| | | SDh INGFp | 15-30 |

Exemple 3: Étude d'oligonucléotdes antisens de l'invention comprenant une région complémentaire de l'oncogène *EWS-Fli1* du Sarcome d'Ewing.

1) Choix des oligonucléotides antisens selon l'invention.

*EWS-Fli1* est le gène oncogène du sarcome de Ewing (tumeur solide et hématologique de l'enfant) et des tumeurs primitives neuroectodermales.

Pour inhiber le gène *EWS-Fli1,* l'ARN a été choisi en tant que cible, car il produit une protéine chimérique EWS-Fli1. Cette protéine, responsable de la maladie provoque le développement du sarcome de Ewing (Tanaka, K., Iwakuma, T., Harimaya, K., Sato, H., Iwamoto, Y. J. Clin. Invest. 1997, 239-247; Toreetsky, J.A., Connell, Y., Neckers, L., Bhat, K. J. Neuro-Oncology. 1997, 31, 9-16). L'ARN est constitué par moitié de la recombinaison 5' *EWS* (gène du chromosome 22) avec la moitié de la recombinaison 3' *(Fli1* gène du chromosome 11), c'est une translocation des chromosomes 22 et 11 pour réaliser une chimère 22/11.

L'oncogène protéique contient 1500 bases. Pour inhiber l'oncogène il est nécessaire d'utiliser des oligonucleotides antisens centrés sur le "break point" de l'oncogène de fusion. Dans les articles de l'art antérieur (Tanaka, K., Iwakuma, T., Harimaya, K., Sato, H., Iwamoto, Y. J. Clin. Invest. 1997, 239-247; Toreetsky, J.A., Connell, Y., Neckers, L., Bhat, K. J. Neuro-Oncology. 1997, 31, 9-16), les auteurs identifient des oligonucleotides phosphorothioates capables d'inhiber l'oncogène chimérique in vitro et in vivo : ces oligonucléotides sont linéaires.

Dans le cadre de la présente invention, des oligonucleotides phosphorothioates antisens, mais structurés ici par une structure secondaire ont été préparés, dans le but d'une part de protéger l'antisens de la dégradation et d'autre part de faciliter son interaction avec la cible et son efficacité *in vitro* et *in vivo*.

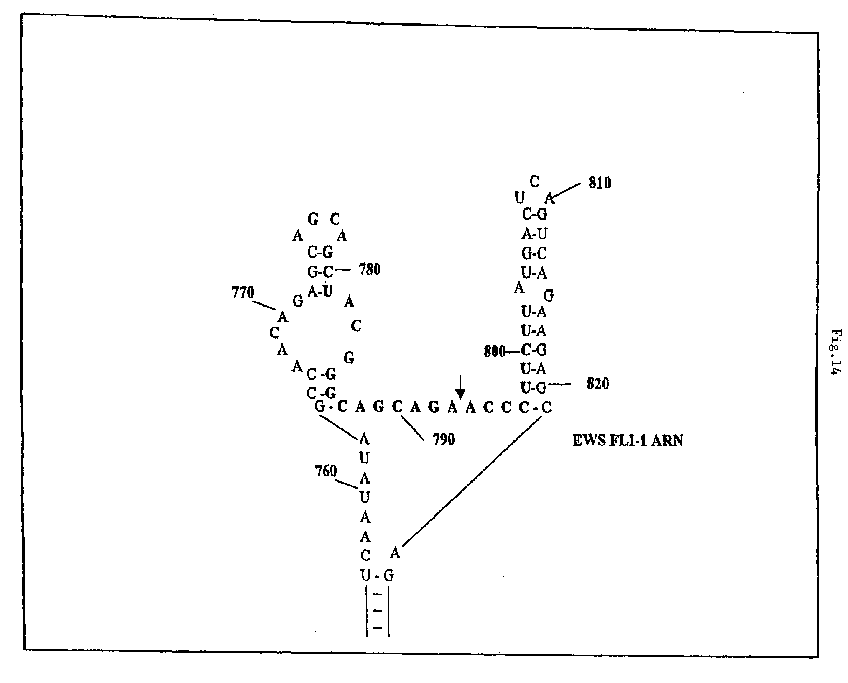

Pour choisir la structure secondaire la plus adaptée à la cible, la structure secondaire de cette cible a été calculée à l'aide du programme "RNA Structure 3.21". La figure 14 en annexe représente la structure de cette cible. Toutes les séquences du RNA *EWS-Fli1* ont été analysées et 6 structures secondaires ont conservé les structures de la cible. Les oligonucleotides antisens structurés selon l'invention choisis contre cette cible sont deux types.

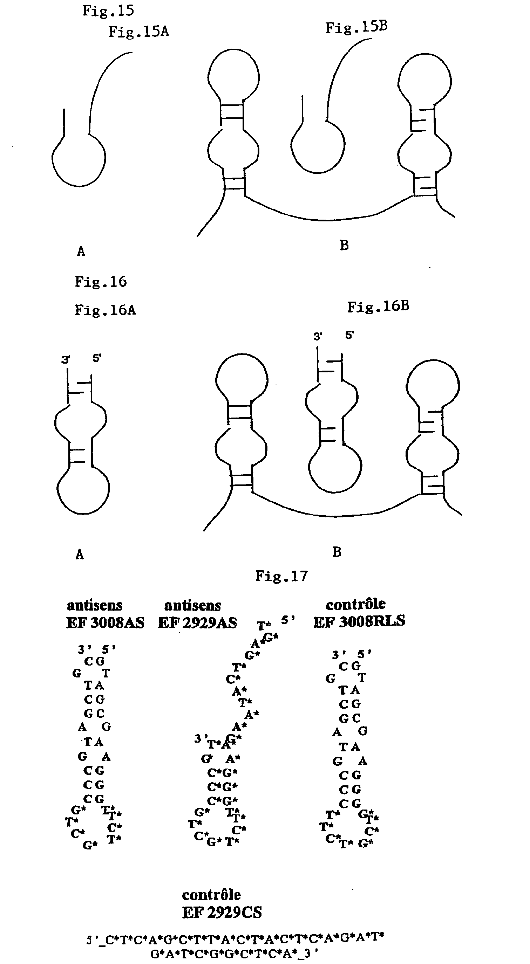

Un premier type désigné EF 2929AS dont la structure est représenté à la figure 15 A en annexe a été préparé. Celui n'utilise aucun élément rajouté vis à vis de la cible pour réaliser la structure secondaire. La figure 15 B montre les interactions avec la cible. Il forme en 3' une structure avec une boucle qui interagit avec la cible et a également en 5' une autre interaction avec une boucle de la cible. L'oligonucléotide structuré EF 2929AS se présente donc comme une succession complémentaire de la cible. Avec cette nouvelle conception, les interactions avec la cible sont multiples, d'une part en 3'avec un boucle et d'autre part en 5' « simple brin » sur une boucle de la cible elle même.

Un deuxième type d'oligonucléotide antisens selon l'invention dont la structure est représenté à la figure 16 A en annexe a été préparé en rajoutant une structure secondaire supplémentaire à chaque extrémité 3' et 5'. L'interaction se fait à la fois avec la structure secondaire en interne et avec la cible en externe à plusieurs niveaux. La figure 16 B montre les interactions avec la cible.

Ainsi, un oligonucléotide antisens structuré a été préparé avec un seul élément de structure secondaire rajouté en 5' désigné EF 3008AS. Comme l'oligonucléotide EF 2929AS, l'oligonucléotide EF 3008AS a été conçu pour, grâce à sa structure secondaire, interagir non seulement au sein de l'oligonucléotide structuré mais également d'avoir des interactions multiples à plusieurs niveaux au sein de la cible.

Les différents oligonucléotides structurés réalisés ont été testés sur le plan de la protection de l'activité in vitro et in vivo une administration avec des nanoparticules (Transdrug® ) ou un vecteur appelé Superfect® a également été étudiée sur le plan de la protection et de l'efficacité.

La séquence des oligonucléotides désignés EF 2929AS et 3008AS et des contrôles correspondant sont représentés à la figure 17 où * représente les groupes phosphorothioates.

La séquence de l'oligonucléotide EF 2929AS est représenté dans la liste de séquence en annexe sous le numéro SEQ ID NO.1 et la séquence de l'oligonucléotide 3008AS est représenté dans la liste de séquence en annexe sous le numéro SEQ ID NO.2.

2) <u>Résultats.</u>

a) <u>Résistance à la dégradation.</u>

La capacité des Oligonucléotides EF 2929AS et EF 3008AS, réalisés avec une structure secondaire suivant l'invention pour résister à l'hydrolyse enzymatique, a été étudiée dans du DMEM supplémenté avec 10% de sérum de veau nouveau-né (appelé NCS: newborn calf sérum heat inactivated) et du sérum humain (MH). Afin d'éviter le clivage du phosphate marqué au ($^{32}$P), des oligonucléotides avec un phosphate protégé en 5' par un résidu éthyl, ont été utilisés pour cette étude.

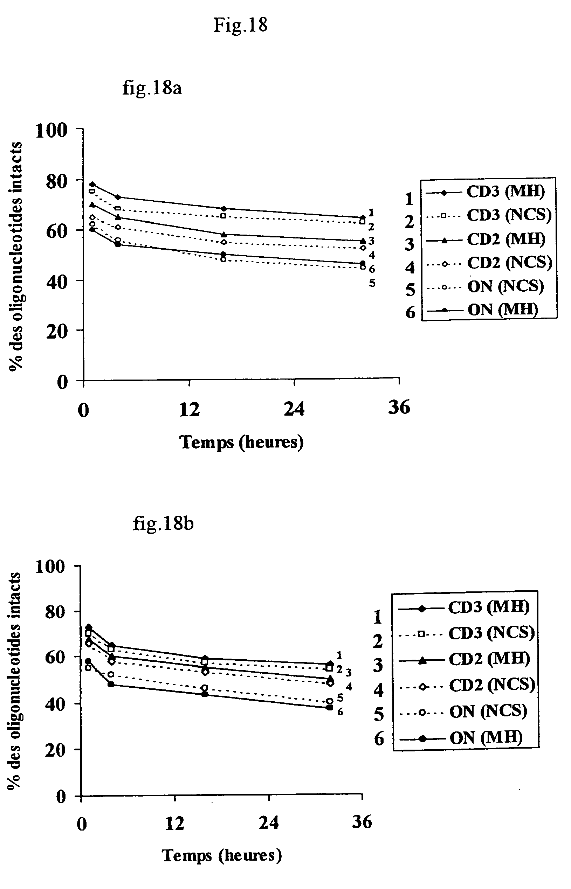

La figure 18 (en A : 3008AS) et (en B : 2929AS) concerne l'analyse de la dégradation de l'oligonucléotide dans le milieu de culture contenant du sérum de veau nouveau-né (NCS) ou du sérum humain (MH), elle montre que la structure secondaire augmente significativement la résistance des oligonucléotides aux nucléases.

Deux nanoparticules PIHCA (Transdrug® ) d'une taille de 65 nm (CD2) et 100 nm (CD3) ont été réalisées suivant le brevet Monza (BioAlliance), une préparation d'oligonucléotides adsorbés sur ces nanoparticules a été réalisée. L'influence de cette préparation avec des nanoparticules sur la stabilité de l'Oligonucléotide dans le DMEM supplémenté avec 10% de sérum de veau nouveau-né, NCS, et du sérum humain, MH, été étudiée. Comme on peut l'observer dans la figure 18, les complexes formés par les oligonucléotides adsorbés avec des nanoparticules présentent une plus forte résistance à la dégradation nucléotidique que les oligonucléotides seuls.

b) <u>Efficacité biologique.</u>

La méthodes de mesure de l'inhibition de prolifération cellulaire est la suivante :

J1: 100.000 cellules EWS/Fli1 et 3T3 par puits sont réparties dans des plaques 6 puits (2 puits pour un point).
J2: Les cellules sont lavées au PBS et 600 μl de 10% d'une solution de sérum de veau nouveau-né (NCS) sont ajoutés. Les complexes ODNs sont réalisés avec SuperFect™ ou Transdrug® (BioAlliance Pharma) dilués dans 200 μl de milieu sans NCS ni antibiotiques, sont ajoutés sur les cellules. Du milieu complet est ajouté dans chaque puit pour avoir un volume final de 800 μl.
J3: Après incubation avec les ODNs (16 h après transfection), les cellules sont lavées avec du PBS et 1ml de 10% d'une solution NCS est ajouté. Le soir les cellules sont lavées avec du PBS et 600 $\mu$l de 10% d'une solution NCS sont ajoutés.

Les ODNs avec SuperFect™ dilués dans 200 $\mu$l de milieu sans NCS ni antibiotiques, sont ajoutés sur les cellules. Du milieu complet est ajouté dans chaque puit pour avoir un volume final de 800 $\mu$l.

J4: Après incubation avec les ODNs (16 h après la transfection), les cellules sont lavées avec du PBS et 400 $\mu$l de la trypsine sont ajoutés. La croissance des cellules est ensuite calculée.

Un effet d'inhibition des oligonucleotides a calculé comme

$$I \ (AO) \ = [N_1(AO) \ - \ N_0(AO)]/N,$$

ou

N - nombre de cellules sans oligonucleotides
$N_0(AO)$ - nombre cellules avant transfection
$N_1(AO)$ - nombre cellules 2 jours après la première transfection.

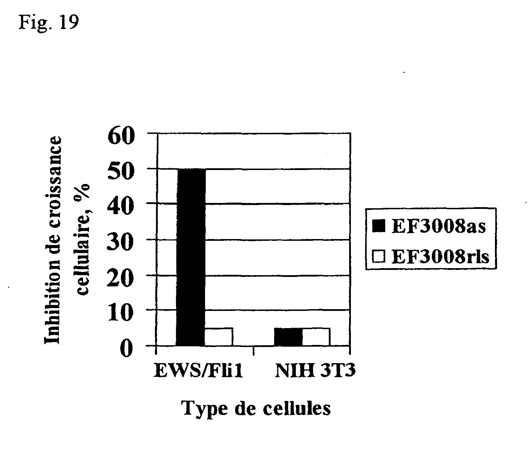

L'inhibition de la prolifération cellulaire a été réalisée sur des cellules 3T3 exprimant EWS/fli1, un contrôle avec des cellules 3T3 normales a permis de mesurer l'impact de l'inhibition de la croissance cellulaire. Ces résultats sont reportés sur la figure 19 en annexe. Il apparaît clairement que l'oligonucléotide structuré antisens EF3008AS est actif sur ce critère, comparé au contrôle négatif de l'oligonucléotide structuré qui a une séquence inversée EF3008RLS. On constate que EF3008AS induit 50% d'inhibition de la croissance cellulaire.

c) <u>Modèle in vivo du sarcome de EWING.</u>

L'étude a été réalisée sur des souris transgeniques (souris nude, modèle développé au sein de l'équipe de C. Auclair F. Subra) qui expriment EWS/Fli1 et qui présentent la maladie sous forme de tumeur (Sarcome de Ewing), cette tumeur palpable apparaît entre 14 à 28 jours suivant l'injection de cellules tumorales.

Le protocole décrit ci-dessous a été mis en oeuvre.

Des souris nudes mâles âgées de 6 semaines sont préparées, elles reçoivent des cellules EWS/Fli1 à partir de cultures cellulaires qui sont re-suspendues à raison de 5 10 puissance 6 cellules par ml dans du PBS, 200 micro litres de cette solution sont injectées dans chaque souris (groupe de 3 souris par type de traitement testé). 14 à 28 jours après l'inoculation, les traitements sont injectés en intratumoral dans une tumeur qui a au moins une taille de 2 à 4 mm3, puis 4 autres injections sont réalisées au jour 5, 8 , 12 , 15 après la première injection. Les animaux sont sacrifiés 21 jours, après la dernière injection.

Le volume tumoral est apprécié durant l'expérimentation par deux mesures perpendiculaires (longueur L et largeur W et le calcul retient LW2/ 2).

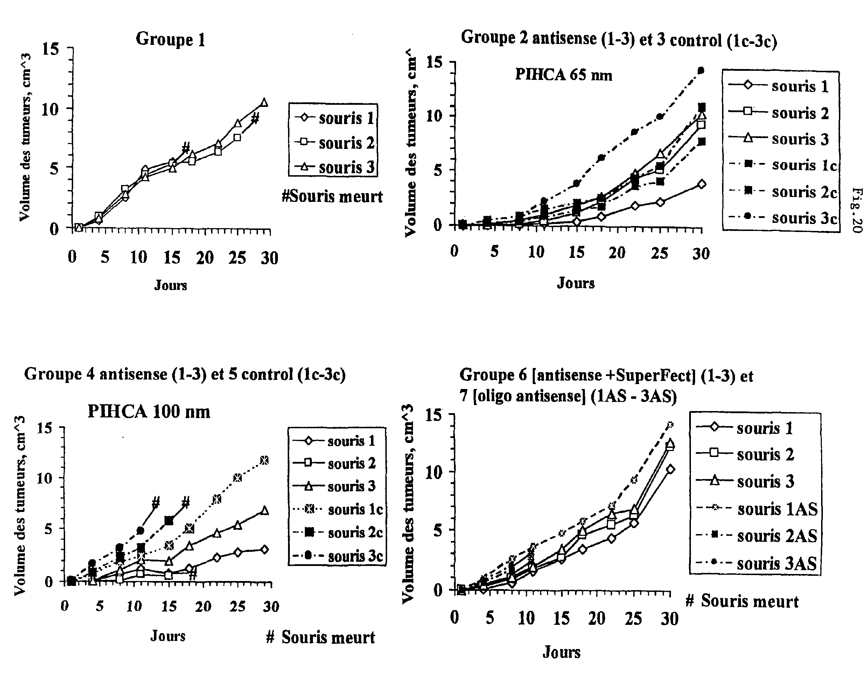

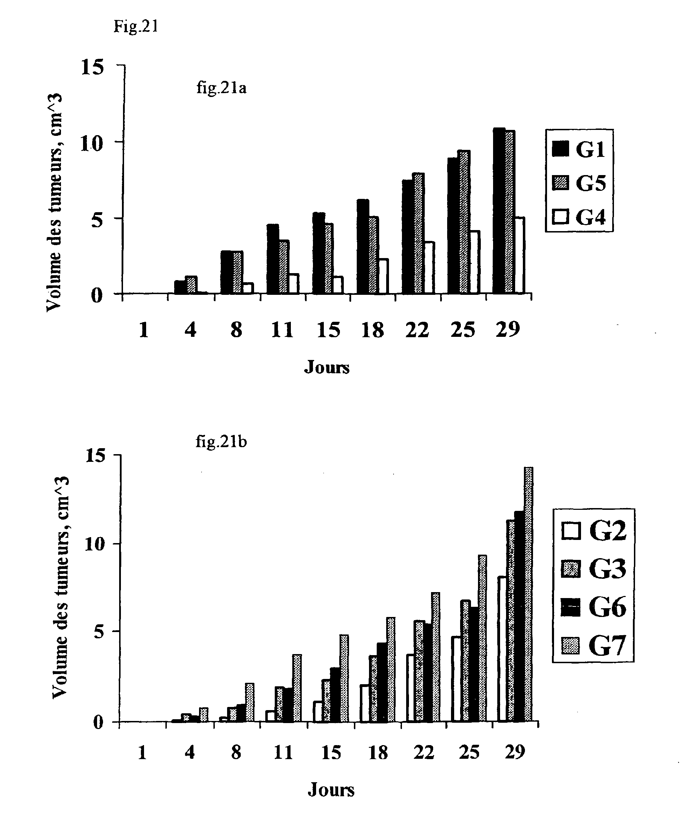

Les groupes de traitement rapportés sur les figures 20 et 21 sont les suivants :

Groupe 1 : souris témoins sans injection d'oligonucléotides
Groupe 2 : souris 1 2 3 oligonucléotides antisens structurés 100 micro litres de PBS contenant 20 micro grammes d'oligonucléotides EF 3008 AS adsorbés sur des nanoparticules (50 micro grammes/ml) et 50 micromoles de CTAB. Taille des nanoparticules de 65 nanomètres.
Groupe 3 Souris 1c,2c,3c oligonucléotides contrôle négatif EF 3008 RLS dans les mêmes conditions avec des nanoparticules de 65 nanomètres.
Groupe 4 : souris 1 2 3 oligonucléotides antisens structurés 100 micro litres de PBS contenant 20 micro grammes d'oligonucléotides EF 3008 AS adsorbés sur des nanoparticules (50 micro grammes/ml) et 50 micromoles grammes de CTAB. Taille des nanoparticules de 100 nanomètres.
Groupe 5 Souris 1c, 2c, 3c oligonucléotides contrôle négatif EF 3008 RLS dans les mêmes conditions avec des nanoparticules de 100 nanomètres.
Groupe 6 : souris 1 2 3 oligonucléotides antisens structurés 100 micro litres de PBS contenant 20 micro grammes d'oligonucléotides EF 3008 AS injectés avec superfect ™54 microgrammes
Groupe 7 Souris 1AS, 2AS, 3AS oligonucléotides contrôle négatif EF 3008 RLS dans les mêmes conditions sans superfect™.

On observe une efficacité *in vivo* avec une stabilisation de la croissance tumorale par l'injection intratumorale d'oligonucléotides structurés suivant l'invention en comparaison avec des témoins négatifs, cet effet est également observé lorsque ceux ci sont administrés avec un vecteur classiquement utilisé ou en utilisant des nanoparticules de taille différentes pour faciliter l'entrée intracellulaire des oligonucléotides.

REFERENCES BIBLIOGRAPHIQUES

1. Curcio L.D.; Bouffard D.Y.; Scanlon K.J. Pharmacol. Ther., **1997**, 74, 317-332.

2. Wyngaarden J.; Potts J.; Cotter F.; Martin R.R.; Mehta V.; Agrawal S.; Eckstein F.; Levin A.; Black L.; Cole R.; Crooke S.; Kreig A.; Diasio R.; Gait M.J. ; Nature Biotech., **1997**,15, 519-524.

3. Agrawal S ; Trends Biotech., **1996**, 14, 376-387.

4. Agrawal S. ; Prospect for Antisense Nucleic Acid Therapy of Cancer and AIDS, **1991**, 143-158

5. Leiter J.M.E.; Agrawal S.; Palese P.; Zamecnik P.C. ; Proc. Natl. Acad. Sci. U.S.A., **1990**, 87, 3430-3434

6. Pagano J.S.; Jimenez G.; Sung N.S.; Raab-Traub N.; Lin J.-C. ; Antisense Strategies. R., **1992**, 107-116

7. Steele C.; Cowsert L.M.; Shillitoe E.J. ; Cancer Res., **1993**, 53, 2330-2337

8. Storey A.; Oates D.; Banks L.; Crawford L.; Crook T. ; Nucl. Acids. Res., **1991**, 19(15), 4109-4114

9. Kulka M.; Smith C.C.; Aurelian L.; Fishelevich R.; Meade K.; Miller P.; TS'O P.O.P. ; Proc. Natl. Acad. Sci. U.S.A., **1989**, 86, 6868-6872

10. Hoke, G.D.; Draper, K.; Freier, S.M.; Gonzalez. C.; Driver, V.B.; Zounes, M.C.; Ecker, D.J. Nucleic Acids Res., **1991**, 1 9, 5743-5748.

11. Wickstrom E. ; J. Biochem. Biophys. Methods, **1986**, 13, 97-102.

12. Eder P.S.; Devine R.J.; Dagle J.M.: Walder J.A. ; Antisense Res. Dev., **1991**, 1, 141-151.

13. Stephenson M.L.; Zamecnik P.C. ; ; Proc. Natl. Acad. Sci. U.S.A., **1978**, 75, 285-288

14. Bonfils E.; Depierreux C.; Midoux P.; Thoung N.T.; Monsigny M.; Roche A.C. ; Nucl. Acids. Res., **1992**, 20(17), 4621-4629

15. Wagner E.; Cotten M.; Mechtler K.; Kirlappos H.; Birnstiel M.L. ; Bioconjugate Chem., **1991**, 2, 226-231

16. Leonetti J.P.; Degols G.; Lebleu B. ; Bioconjugate Chem., **1993**, 1, 149-153

17. Perrouault L.; Asseline U.; Rivalle C.; Thoung N.T.; Bisagni E.; Giovannangeli C.; Le Doan T.; Héline C. ; Nature, **1990**, 344, 358-360

18. Gamper H.B.; Reed M.W.; Cox T.; Virosco J.S.; Adams A.D.; Gall A.A.; Scholer J.K.; Meyer R.B.J. ; Nucl. Acids. Res., **1993**, 21(1), 145-150

19. Ortigao J.F.R.; Rösch H.; Selter H.; Frôhlich A.; Lorenz A.; Montenarh M.; Seliger H. ; Antisense Res. Dev., **1992**, 2, 129-146

20. Schaw, J.-P.; Kent K.; Bird J.; Fishback J.; Froechler B. ; Nucleic Acids Res., **1991**, 19, 747-750.

21. Uhmaln E.; Peyman A. ; Chem. Reviews, **1990**, 90, 544-584.

22. Stein C.A.; Cohen J.S. ; Cancer Res., **1998**, 48, 2659-2668.

23. Nielsen P.E. ; Ann. Rev. Biophys. Biomol. Struct., **1995**, 24, 167-183.

24. Morvan F.; Porumb M.; Degols G.; Lefebvre I.; Pompon A.; Sproat B.S.; Rayner B.; Malvy C.; Lebleu B.; Imbach J.-L. ; J. Med. Chem., **1993**, 36, 280-283.

25. Stein C.A. ; Chemistry & Biology, **1996**, 3, 319-323.

26. Agrawal S.; Zhao Q.; Jiang Z.; Oliver C.; Giles H.; Heath J.; Serota D. ; Antisense & Nucl. Acid Drug Dev., **1997**, 7, 575-584.

27. Branch A.D. TiBS, **1998**, 23, 45-50.

28. Jansen B.; Wadl H.; Inoue S.A.; Trulzsch B.; Selzer E.; Duchene M.; Fichler H.; Wolf K; Pehamberger H. ; Antisense Res. Dev., **1995**, 5, 271-277.

29. Henry S.P.; Zuckerman J.E.; Rojko J.; Hall W.S.; Harman R.J.; Kitchen D.; Crooke S.T. Anti-Cancer Drug Design, **1997**, 12, 1-4.

30. Khan I.M.; Coulson M. ; Nucl. Acids Res., **1993**, 21, 2957-2958.

31. Poddevin B.; Meguenni S.; Elias I.; Vasseur M.; Blumenfeld M. ,Antisense Res. Dev., **1994**, 4, 147-154.

32. Yoshizawa S.; Ueda T.; Ishido Y.; Miura K-i.; Watanabe K.; Hirao I. ; Nucl. Acids Res., **1994**, 22, 2217-2221.

33. Tang J.Y.; Temsamani J.; Agrawal S. ; Nucl. Acids Res., **1993**, 21, 2739-3735.

34. Kuwasaki T.; Hosono K.; Takai K.; Ushijima K.; Nakashima H.; Saito T.; Yamamoto N.; Takaku H. ; Biochem. Biophys. Res. Com., **1996**, 228, 623-631.

35. Barker R.H.; Metelev V.; Coakley A.; Zamecnik P. ; Exp. Parasitology, **1998**, 88, 51-59.

36. Xodo L.E; Manzini G.; Quadrifoglio F.; van der Marel G.A.; van Boom J.H. ; Biochimie, **1989**, 71, 793-803.

37. Petersheim M.; Turner D.H. ; Biochem., **1982**, 22, 256-263.

38. Capaccioli S.; Di Pasquale G.; Mini E.; Mazzei T.; Quattrone A. ; Biolochem. and Biophys. Res. Com, **1993,**197, 818-825.

39. Chavany C.; Saison-Behmoaras T.; Le Doan T.; Puiseux F.; Couvreur P.; Helene C. ; Pharm. Res., **1994**,11, 1370-1378.

40. Delong R.; Stephenson K.; Loftus T.; Fisher M.; Alahari S.; Nolting A.; Juliano R. L. ; .J. Pharm. Sci., **1997**, 86, 762-764.

41. Morris M. C.; Vidal P.; Chaloin L.; Heitz F.; Divita G. ; Nucleic Acids Res., **1997**, 25, 2730-2736.

42. Poxon S. W.; Mitchell P. M.; Liang E.; Hughes J. A. ; Drug Delivery, **1996**, 3, 255-261.

43. Couch R.J. ; New Biologist, **1990**, 2, 771-777.

44. Kukowska-Latallo J.F.; Bielinska A.U.; Jonson J.; Spindler R.; Tomalia D.A.; Baker J.R. ; Proc. Natl. Acad. Sci. USA, **1996**, 93, 4897-4902.

45. Bielinska A.; Kukowska-Latallo J.F.; Jonson J.; Tomalia D.A.; Baker J.R. ; Nucleic Acids Res., **1996**, 24, 2176-2182.

46. Tang M.X.; Redemann C.T.; Szoka F.C. ; Bioconjugate Chem., **1996**, 7, 703-714.

**Revendications**

1. Utilisation à titre de principe actif de l'un au moins des oligonucléotides représentés dans la liste de séquence sous les numéros SEQ ID NO.1 ou SEQ ID NO.2 dans la préparation d'une composition pharmaceutique pour le traitement du sarcome d'EWING.

2. Utilisation selon la revendication 1, **caractérisée en ce que** les oligonucléotides sont identiques ou différents, libres, encapsulés, couplés, conjugués à des substances diverses, associés dans ladite composition à un véhicule pharmaceutiquement acceptable.

**Claims**

1. Use of at least one of the oligonucleotides shown in the sequence list under numbers SEQ ID No. 1 or SEQ ID No. 2 as an active constituent in the preparation of a pharmaceutical composition for the treatment of EWING's sarcoma.

2. Use according to claim 1, **characterised in that** the oligonucleotides are identical or different, free, encapsulated, bond, conjugated to miscellaneous substances, associated with a pharmaceutically acceptable vehicle in the said composition.

**Patentansprüche**

1. Einsatz zumindest eines der Oligonucleotide, die in der Folgeliste unter den Nummern SEQ ID NO. 1 oder SEQ ID NO. 2 aufgeführt werden, als Wirkstoff zur Herstellung einer pharmazeutischen Rezeptur zur Behandlung eines EWING-Sarkoms.

2. Einsatz gemäß Anspruch 1, **dadurch gekennzeichnet, dass** die Oligonucleotide identisch oder unterschiedlich, frei, eingekapselt, gekoppelt, mit anderen unterschiedlichen Substanzen vereinigt, in der besagten Rezeptur mit einem aus pharmazeutischer Sicht annehmbaren Arzneistoffträger verbunden sind.

FIG.1

FIG.2

FIG.3

FIG.4

FIG.5

FIG.6

Fig.7

## SEQUENCES CIBLES

**D**  5'-d (CCAGC<u>AGAATCGACACATGGCGTGTTCA</u>ACGCT)-3'

**R**  5'-r (CCAGC<u>AGAAUCGACACAUGGCGUGUUCA</u>ACGCU)-3'

## OLIGODEOXYNUCLEOTIDES ANTISENS

**21L**  5'-TGAACACGCCATGTCGATTCT-3'

**55L**  5'-T$_2$ACT$_3$CT$_5$GCGTTGAACACGCCATGTCGATTCTT$_5$CT$_5$C$_6$-3'

**21PS**  5'-T*G*AACACGCCATGTCGATTC*T-3'

**H6**  5'-TGAACACGCCATGTCGATTCT$_T$
       3'-CTAAGA$^T$

**H8**  5'-TGAACACGCCATGTCGATTCT$_T$
       3'-AGCTAAGA$^T$

**H10**  5'-TGAACACGCCATGTCGATTCT$_T$
        3'-ACAGCTAAGA$^T$

**Dh6**  $_C$TGAACACGCCATGTCGATTCT$_T$
        $^T$ACTTGT-5'     3'-CTAAGA$^T$

**L8**  5'-GCGTA$^T$GAA$^{C}$$^{ACG}$$^{C}$$_C$
       3'-CGCAT$_T$CTT$_A$$_{GCT}$$_G$$^T$$^A$

**L10**  5'-GCGCTTA$^T$GAA$^{C}$$^{ACG}$$^{C}$$_C$
        3'-CGCGAAT$_T$CTT$_A$$_{GCT}$$_G$$^T$$^A$

fig.8a

fig.8b

Fig.9

5'-CCAGCAGAAUCGACAUGGCGUGUUCAACGCU-3'
T<sup>TCTTAGCTGTACCGCACAAGT</sup>-5'
T<sup>AGAATCGACA</sup>-3'

H10

Fig.10

Fig.11

Fig.11b

Fig.11a

Fig.11d

Fig.11c

Fig.12

fig.12b

fig.12a

fig.12d

fig.12c

Fig.13

# Oligonucléotides phosphodiesteres

## Dh INGFp

15-25

## Sh-6 INGFp

15-25

## L4 INGFp

15-25

## L2 INGFp

5-10

## L7 INGFp

10-15

## C7 INGFp (control négatif)

0

# Oligonucléotides phophorothioates

## L2 INGFp 2S

65-80

## L4 INGFp 2S

30-40

## SDh INGFp

15-30

## 21 PS (control négatif)

5'-T*G*AACACGCCATGTCGATTC*T*-3'

0

***: groupement phosphorothioate**

EWS FLI-1 ARN

Fig. 14

Fig.15

Fig.15A

Fig.15B

A

B

Fig.16

Fig.16A

Fig.16B

3'  5'

3'  5'

A

B

Fig.17

**antisens**
**EF 3008AS**

**antisens**
**EF 2929AS**

**contrôle**
**EF 3008RLS**

T* 5'
A G*
G*
T*
C*
A*
T*
A*
A*
3' T* G*
G*  A*
C*G*
C*G*
C*G*
G*    T*T*
T*        C*
C* G* T*

3' 5'
C G
G   T
T A
C G
G C
A   G
T A
G   A
C G
C G
C G
G*   T*T*
T*       C*
C*      T*
G*

3' 5'
C G
G   T
T A
C G
G C
A   G
T A
G   A
C G
C G
C G
T*   G*T*
T*       C*
C*T* G*

**contrôle**
**EF 2929CS**

5'_C*T*C*A*G*C*T*T*A*C*T*A*C*T*C*A*G*A*T*
G*A*T*C*G*G*C*T*C*A*_3'

Fig.18

fig.18a

fig.18b

Fig. 19

Type de cellules

Fig.20

Fig.21

fig.21a

fig.21b